# EUROPEAN PATENT SPECIFICATION

(11) **EP 1 833 995 B1**
(45) Date of publication and mention of the grant of the patent: **02.08.2017**
(21) Application number: 06717583.6
(22) Date of filing: 05.01.2006
(51) Int. Cl.: C12Q 1/68

(54) **IDENTIFICATION OF RNA TARGETS USING HELICASES**
IDENTIFIZIERUNG VON RNA-TARGETS MITTELS HELIKASEN
IDENTIFICATION DE CIBLES D'ARN AU MOYEN D'HELICASES

(30) Priority: 05.01.2005 US 641419 P
(43) Date of publication of application: 19.09.2007
(73) Proprietor: Biohelix Corporation, Beverly, MA 01915 (US)
(72) Inventor: KONG, Huimin, Wenham, Massachusetts 01984 (US); TANG, Wen, Boston, Massachusetts 02120 (US)
(74) Representative: Gibson, Mark
(86) International application number: PCT/US2006/000406
(87) International publication number: WO 2006/074334

(56) References cited:
- WO-A-93/14217
- WO-A-2004/027025
- DE-A1- 10 314 745
- US-A1- 2005 069 926
- VINCENT MYRIAM ET AL: "Helicase-dependent isothermal DNA amplification." EMBO REPORTS. AUG 2004, vol. 5, no. 8, August 2004 (2004-08), pages 795-800, XP002400120 ISSN: 1469-221X
- AN LIXIN ET AL: "Characterization of a thermostable UvrD helicase and its participation in helicase-dependent amplification." THE JOURNAL OF BIOLOGICAL CHEMISTRY. 12 AUG 2005, vol. 280, no. 32, 12 August 2005 (2005-08-12), pages 28952-28958, XP002400121 ISSN: 0021-9258

## Description

### BACKGROUND

Identification of RNA targets using amplification methods is desirable in applications that include: diagnostic applications for RNA-based pathogens like RNA viruses; and monitoring the levels of gene expression, for example, dysregulation of cancer genes. As the copy number of mRNA transcripts of an expressed gene is generally greater than the gene itself, amplification of RNA targets may greatly enhance the diagnostic sensitivity for the gene and thereby overcome problems caused by inadequate sensitivity in some amplification reactions. RNA amplification techniques are described for example in U.S. Pat. Nos. 5,322,770, 5,624,833, and 6,794,177. These methods generally require a reverse transcriptase step followed by an amplification step. Although RT-PCR is extremely sensitive for detection of RNA targets, the format is similar to PCR reaction itself and requires expensive thermocycler equipment for performance. (See, for example, U.S. Pat. No. 6,794,177).

Amplification of RNA targets may also be performed in one step without temperature cycling in combination with reverse transcription (RT) and an isothermal amplification platform. An example is reverse transcription-strand-displacement amplification (RT-SDA) in which a reverse transcriptase and the SDA platform are used to amplify RNA targets in one isothermal step (U.S. Pat. No. 5,916,779). However, like SDA, RT-SDA requires modified nucleic acids and more than one pair of primers for efficient performance.

Reference may, for example also be made to WO 2004/027025 A, which relates to helicase dependent amplification of nucleic acids, and to US 2005/0069926 A1, which relates to helicase-amplified reverse transcription.

### SUMMARY OF THE INVENTION

An embodiment of the invention includes a method for identifying an RNA molecule that includes: (a) synthesizing a cDNA from the RNA molecule by RT to form an RNA/DNA duplex; (b) at least partially separating the duplex into a cDNA and an RNA using a thermostable helicase; (c) amplifying the cDNA; and (d) characterizing the cDNA to determine the identity of the RNA molecule. In particular examples of the method, RT is achieved by means of a reverse transcriptase or a DNA polymerase with RT activity. In addition or alternatively, amplification of the cDNA may be achieved by helicase-dependent amplification (HDA), loop-mediated isothermal amplification, rolling cycle amplification, strand-displacement amplification (SDA) and polymerase chain reaction (PCR).

In an embodiment of the invention, the RNA in the DNA/RNA duplex can be reused for RT and amplification in a repeating cycle. The entire method can be achieved in a single buffer in a single reaction vessel or alternatively can be achieved using separate buffers for some or all of the steps. For example, steps (a) and (b) can be performed in a single buffer or in different buffers which may differ from the buffer or buffers used for steps (b) and (c).

In an embodiment of the method, a single strand binding protein such as described in U.S. patent application 2004-0058378 is not added in step (b) or (c) above. Accordingly, the method utilizes a helicase in a reaction mixture that is essentially free of a single strand binding protein. The thermostable helicase has an effective activity in the absence of a single strand binding protein.

In an embodiment of the invention, a method is provided for exponentially and selectively amplifying a target nucleic acid that includes: (a) providing single strand templates of the target nucleic acid to be amplified; (b) adding oligonucleotide primers for hybridizing to the templates of step (a); (c) synthesizing an extension product of the oligonucleotide primers which are complementary to the templates, by means of a DNA polymerase to form a duplex; (d) contacting the duplex of step (c) with a thermostable helicase for unwinding the duplex; and (e) repeating steps (b)-(d) to exponentially and selectively amplify the target nucleic acid. In an example of the method, the thermostable helicase is utilized in the absence of a single strand binding protein.

In an embodiment of the invention, a kit is provided that includes a thermostable helicase, a reverse transcriptase, a DNA polymerase and a buffer containing at least three reagents selected from the group consisting of: a magnesium salt, a sodium salt, dNTP, and dATP; the kit containing instructions for use in amplifying a DNA in a reaction that does not include a single strand binding protein. The buffer may for example have a pH in the range of pH 8 - pH 10. In other examples of the kit, the polymerase may be Bst polymerase and/or the thermostable helicase may be Tte-UvrD helicase.

### BRIEF DESCRIPTION OF THE DRAWINGS

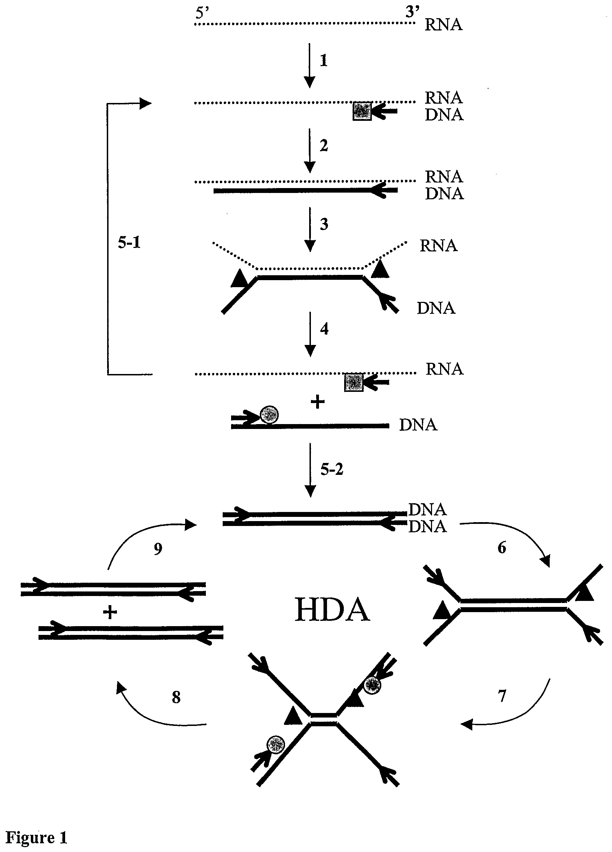

Figure 1 shows a schematic diagram of an RT-HDA reaction.
   Step 1 shows binding a single primer to a target RNA.
   Step 2 shows the synthesis of a first strand cDNA by RT of the target RNA using a reverse transcriptase or polymerase with RT properties.
   Step 3 shows the helicase (black triangles) unwinding the DNA/RNA duplex into at least partial single strand nucleic acids.
   Step 4 shows the addition of at least a second primer so that the first and second primers bind to the boundaries of the nucleic acid sequence of interest so as to permit amplification of the DNA sequence between the primers.
   Step 5-1 shows how displaced single strand RNA or the original target RNA undergoes an RT reaction (repeating steps 1-4).
   Step 5-2 shows how primer binds to single strand DNA (ssDNA) permitting amplification to occur by HDA or other type of amplification.
   Steps 6-9 show stages during the amplification.
   Step 6 shows how the duplex DNA is unwound either by heat or by HDA or other means.
   Step 7 shows primers binding to ssDNA.
   Step 8 shows extension of the DNA polymerases from primers.
   Step 9 shows that the amplified products enter the next round of amplification.
   Dashed lines: RNA; Solid thick lines: DNA; Lines with arrows: primers. Black triangles: helicases; grey rectangles: reverse transcriptases; grey circles: DNA polymerases.

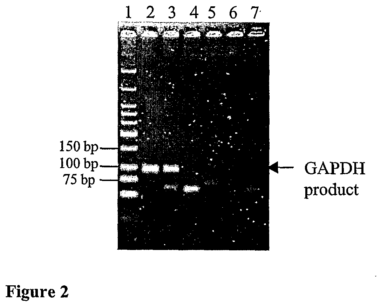

Figure 2 shows the results of gel electrophoresis (2% agarose) of amplified GAPDH RNA in a two-step reaction.
   Two-step RT-HDA reaction was performed in two different reaction tubes. The first strand cDNA synthesis was carried out using ProtoScript^{®} Kit (New England Biolabs, Inc., Ipswich, MA) and 1 µg of human total RNA. The first-strand cDNA synthesized product was then diluted and amplified using HDA in the presence of Tte-UvrD (helicase) and *Bst* DNA polymerase in a total volume of 50 µl. Ten µl of each sample was analyzed on the gel. The percentages of the first-strand cDNA synthesized product varied as indicated in lanes 2-7.
   Lane 1: 200 ng of Low Molecular Weight DNA Ladder (New England Biolabs, Inc., Ipswich, MA);
   Lane 2: 1%;
   Lane 3: 0.1%;
   Lane 4: 0.01%;
   Lane 5: 0.001%;
   Lane 6: 0.0001%;
   Lane 7: 0.

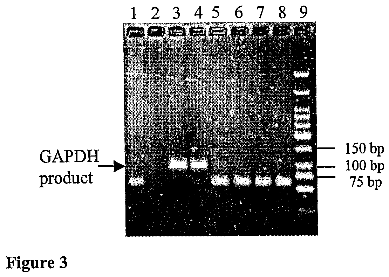

Figure 3 shows the results of gel electrophoresis (2% agarose) of amplified GAPDH RNA starting from different amounts of human total RNA in a two-step reaction.
   Two-step RT-HDA reaction was performed in one reaction tube. RT-HDA was carried out by mixing Tte-UvrD, M-MuLV reverse transcriptase, *Bst* DNA polymerase, and thermophilic HDA (tHDA) buffer in one tube in a total volume of 50 µl, incubated at 42°C for 5 minutes followed by 65°C for 60 minutes. 10 µl of each sample was analyzed on the gel. The amounts of total RNA in lanes 1-8 are as follows:
   Lanes 1, 2, 3: 100 ng;
   Lane 4: 10 ng;
   Lane 5: 1 ng;
   Lane 6: 100 pg;
   Lane 7: 10 pg;
   Lane 8: 0;
   Lane 9: 200 ng of Low Molecular Weight DNA Ladder (New England Biolabs, Inc., Ipswich, MA).
   The first two lanes are from control reactions without reverse transcriptase or helicase respectively.

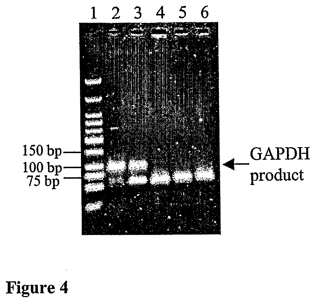

Figure 4 shows the results of gel electrophoresis (2% agarose) of amplified GAPDH RNA starting from different amounts of human total RNA in a one-step reaction.
   One-step RT-HDA reaction was performed in a single reaction vessel. RT-HDA was carried out by mixing Tte-UvrD, SuperScript™ III (Invitrogen, Carlsbad, CA) reverse transcriptase, *Bst* DNA polymerase, and tHDA buffer in one tube with a total volume of 50 µl and incubated at 62.5°C for 60 minutes. 10 ml of each sample was analyzed on the gel. The amounts of total RNA are:
   Lane 1: 200 ng of Low Molecular Weight DNA Ladder (New England Biolabs, Inc., Ipswich, MA).
   Lane 2: 100 ng;
   Lane 3: 10 ng;
   Lane 4: 1 ng;
   Lane 5: 100 pg;
   Lane 6: 0.

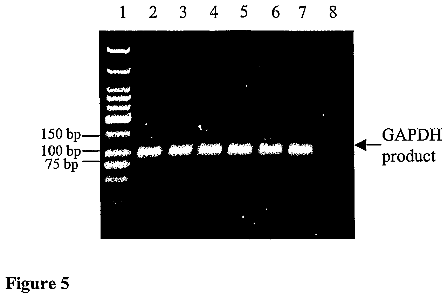

Figure 5 shows the results of gel electrophoresis (2% agarose) of amplified GAPDH RNA starting from different amounts of human total RNA and using a modified one-step RT-HDA reaction.
   RT-HDA was carried out by first denaturing Mix A containing RNA template and a pair of GAPDH specific primers at 95°C for 2 minutes. Mix B containing Tte-UvrD, ThermoScript™ (Invitrogen, CA) reverse transcriptase, *Bst* DNA polymerase was then added into Mix A and incubated at 65°C for 120 minutes. 10 µl was analyzed on the gel. The amounts of total RNA are:
   Lane 1: 250 ng of Low Molecular Weight DNA Ladder (New England Biolabs, Inc., Ipswich, MA).
   Lane 2: 200 ng;
   Lane 3: 20 ng;
   Lane 4: 2 ng;
   Lane 5: 200 pg;
   Lane 6: 20 pg;
   Lane 7: 2 pg;
   Lane 8: 0.

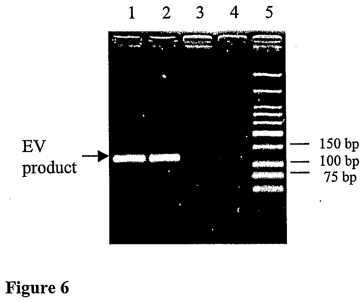

Figure 6 shows the detection of Enterovirus using a modified one-step RT-HDA, varying amounts of Enterovirus RNA and 2% agarose gel electrophoresis. RT-HDA was carried out as described in Figure 5 using a pair of Enterovirus specific primers. 10 µl was analyzed on the gel. The amounts of Enterovirus RNA (EV product) are:
   Lane 1: 4000 copies;
   Lane 2: 400 copies;
   Lane 3: 40 copies;
   Lane 4: 0.
   Lane 5: 250 ng of Low Molecular Weight DNA Ladder (New England Biolabs, Inc., Ipswich, MA).

### DETAILED DESCRIPTION OF THE INVENTION

Present embodiments of the invention combine reverse transcription of RNA with a helicase and a subsequent or concomitant amplification step of the resulting cDNA in a single reaction vessel or in a plurality of reaction vessels. The amplification step is optionally an HDA but may alternatively be a polymerase chain reaction, a loop-mediated isothermal amplification or a rolling circle amplification. RT-HDA may be performed isothermally at a temperature in the range of about 20°C - 75°C, for example in a range between 60°C - 65°C. In an embodiment of the invention, RT-HDA can be performed to detect or quantify RNA where HDA replaces PCR in amplification protocols such as real time RT-HDA, relative RT-HDA, competitive HDA and comparative RT-HDA.

RNA can be amplified in a reaction or a series of reactions that include RT, thermostable helicase dependent denaturation and amplification. Where the reaction occurs in a single reaction vessel, it is desirable to utilize a single buffer. This constraint is not applicable when RNA is amplified in a series of reactions that occur in different reaction vessels. An advantage of conducting the reactions in a single reaction vessel is that cDNA copies of an RNA target sequence act as a template for DNA amplification at the same time as more cDNA is generated from RNA liberated from the DNA/RNA duplex by RT.

In one embodiment of the invention, the amplification process is a helicase-dependent amplification (see U.S. patent application 2004/0058378). In this approach, amplification can occur in the presence of a helicase preparation, which includes at least one helicase, an energy source such as nucleotide triphosphates and a single strand binding protein or a thermostable helicase in the absence of a single strand binding protein. The helicase preparation is combined with a DNA polymerase, deoxyribonucleotides and primers to give rise to HDA. Where a thermostable helicase is used, a helicase preparation may not be required. The thermostable helicase may not require a single strand binding protein but can be combined with a polymerase, deoxyribonucleotides and primers to affect HDA (see Examples 1-5).

Examples of RNA targets include any RNA obtained from a cell or a virus such as messenger RNA, transfer RNA, ribosomal RNA, double-stranded RNA or other RNAs that occur in cells or viruses.

A reverse transcriptase suitable for use in embodiments of the present method includes any commercially available enzyme used for reverse transcriptase such as M-MuLV, Avian Myeloblastosis Virus (AMV) reverse transcriptase, a thermostable reverse transcriptase such as (SuperScript™, ThermoScript™ (Invitrogen, Carlsbad, CA), or Transcriptor (Roche, Basel, Switzerland) reverse transcriptase or a polymerase with reverse transcriptase activity such as Tth polymerase.

Helicases for use in embodiments of the method are enzymes capable of unwinding double-stranded DNA under normal (mesophilic) or elevated temperatures (thermostable). Helicases are abundant in nature and there is an extensive literature on their properties. They have been identified for prokaryotes, eukaryotes and archaea and like other enzymes, some are thermostable (preferred reaction temperature above about 60°C) while others are thermolabile (preferred reaction temperature between about 20°C - 37°C). An advantage of thermostable helicases such as Tte-UvrD helicase is that they do not require a single strand binding protein, which is required by a thermolabile helicase. While the number of examples that could be provided is extensive, Tte-UVrD helicase is representative of thermostable helicases in general and teaches a person of ordinary skill in the art how to perform the invention. Examples of thermolabile helicases include UvrD-like helicases such as *E. coli* UvrD helicase, RecBCD helicase, and T7 gene 4 helicase and thermostable helicases such as Tte-UvrD helicase (US patent application 20040058378).

Examples of a suitable DNA polymerase include Klenow fragment of *E. coli* DNA polymerase I, T7 DNA polymerase (Sequenase, USB Corporation, Cleveland, Ohio), Vent^{®} DNA polymerase, Vent^{®} (exo⁻) DNA polymerase (New England Biolabs, Inc., Ipswich, MA), Bst DNA polymerase large fragment, and Phusion DNA polymerase (Finnzymes, Espoo, Finland). Preferably, the DNA polymerase lacks 5' to 3' exonuclease activity and possesses strand displacement activity.

The reverse transcriptase, helicase induced denaturation and optionally an HDA can occur in a single reaction vessel in a single buffer. In one embodiment, the buffer contains 20 mM Tris-HCl, pH 8.3 - 8.8, contains 3 - 5 mM MgCl2, 10 mM KCl, 20 - 40 mM NaCl, 0.4 mM dNTP, and 3 mM dATP or ATP.

The temperature of the reaction is preferably biased towards the optimal temperature for the helicase reaction. However, if the reverse transcriptase is heat sensitive, the temperature can be modified accordingly.

Performing RT-HDA in a single reaction vessel is preferable. In such system, cDNA copies are generated and amplified from a RNA target concurrently using a helicase, a reverse transcriptase, and a DNA polymerase. The first strand cDNA is first synthesized by a reverse transcriptase (SuperScript™, ThermoScript™ (Invitrogen, Carlsbad, CA), or Transcriptor (Roche, Basel, Switzerland) reverse transcriptase when the RT and amplification occurs in a single reaction vessel (FIG. 1, steps 1 & 2). The RNA-DNA duplex from the RT is at least partially unwound by helicases generating single-stranded RNA and DNA templates (FIG. 1, steps 3 & 4). In an embodiment of the invention, the helicase unwinds the full length of the RNA/DNA duplex. The single-stranded RNA enters a next round of RT reaction (FIG. 1, step 5-1) generating more first strand cDNA. The ssDNA is converted into double-stranded DNA by the DNA polymerase (FIG. 1, step 5-2) and amplified concurrently in the HDA reaction (FIG. 1, steps 6 - 9). This process repeats itself to achieve exponential amplification of the RNA target sequence (FIG. 1). Helicases that unwind both RNA-DNA dupexes and DNA duplexes are preferred in reactions that occur in a single reaction vessel. These helicases include a UvrD helicase or homolog thereof, for example a thermostable Tte-UvrD helicase.

Alternatively, the RT-HDA can be carried out at two different reactions in the same or different reaction vessels. For example, the RT may be executed at a preferred temperature for the selected reverse transcriptase and the amplification may be preformed at a preferred temperature for the helicase reaction. An example of a two phase reaction involves making cDNA copies using M-MuLV reverse transcriptase at for example 42°C (37°C - 50°C for RT) for about 3 - 10 minutes, more particularly 5 minutes, in a first step followed by amplification in a second step using thermophilic HDA reaction at about 62°C - 66°C for tHDA, more particularly, about 65°C for about 60 - 120 minutes.

Where two different buffer systems are used for RT-HDA, (one for RT and the other for the amplification step), the first strand cDNA is synthesized by a reverse transcriptase in the presence of either an oligo-dT primer or a sequence-specific primer complimentary to the target RNA sequence. In an embodiment of the invention; the RT buffer in a two-phase method is 50 mM Tris-HCl, pH 8.3, 75 mM KCl, 3 mM MgCl₂, 10 mM DTT. AMV: 50 mM Tris acetate, pH 8.4, 75 mM potassium acetate, 8 mM magnesium acetate, 10 mM DTT, dNTPs, and target RNA. Aliquots of cDNA copies from the RT reaction are then transferred into a second reaction buffer and are subsequently amplified in the presence of a helicase and a polymerase.

The cDNA product can be characterized so as to Identify the original RNA sample by methods that are well known in the art such as sequencing and database comparisons, hybridization involving for example DNA arrays such as provided on a chip by Nanogen, San Diego, CA and any suitable probe technology including the use of Molecular Beacon and Taqman® technology (Applied Biosystems, Foster City, CA).

### EXAMPLES

### Example I: two-reaction vessel/two-buffer RT-HDA

In this example, human total RNA was used as nucleic acid substrate and was first converted to a single stranded cDNA product using the ProtoScript^{®} Kit from New England Biolabs (New England Biolabs, Inc., Ipswich, MA).

The reaction was set up by combining:
1 µl human total RNA (1 µg/µl)
2 µl primer dT₂₃VN (50 µM, New England Biolabs, Inc., Ipswich, MA)
4 µl dNTP (2.5 mM)
9 µl nuclease-free H₂O
and started by incubated at 70°C for 5 min, then kept on ice.

The following reagents were then added to the reaction tube:
2 µl 10x RT buffer (New England Biolabs, Inc., Ipswich, MA)
1 µl RNase inhibitor (10 units/µl)
1 µl M-MuLV reverse transcriptase (25 units/µl)

The RT reaction was incubated at 42°C for 1 hr, followed by 95°C for 5 min. 1 µl of RNase H (2 units/µl) was then added and the RT reaction was further incubated at 37°C for 20 min, followed by 95°C for 5 min. The RT product was then diluted into 50 µl with dH₂O. A serial dilution of this diluted RT product was then prepared. In each time, 5 µl of the previously diluted RT product was further diluted into 50 µl with dH₂O. In the subsequent tHDA reaction, a pair of primers, GAPDF1 and GAPDR1, specifically targeting the GAPDH gene was used for amplification. The tHDA reactions were started by combining the following components in reaction mixture A with a total volume of 25 µl:
2.5 µl 10X Mg-free tHDA buffer (see below*)
5 µl of serial diluted RT product (0.0001% to 1% of RT product)
0.5 µl 10 µM Primer GAPDF1 (SEQ ID NO:1)
0.5 µl 10 µM Primer GAPDR1 (SEQ ID NO:2)
16.5 µl dH₂O
25 µl of reaction mixture B was also prepared by mixing:
2.5 µl 10X Mg-free tHDA buffer (see below*)
1.75 µl 100 mM MgSO₄
2 µl 500 mM NaCl
2 µl 10 mM dNTP
1.5 µl 100 mM dATP
2.5 µl Bst DNA Polymerase, LF (8 units/µl; New England Biolabs, Inc., Ipswich, MA)
1 µl Tte-UvrD helicase (100 ng/µl)
11.75 µl dH₂O
*10X Mg-free tHDA buffer contains 100 mM KCl,
100 mM (NH₄)₂SO₄, 200 mM Tris-HCl (pH 8.8 at 25°C), 1 % Triton X-100.

The reaction mixture A was heated for 2 min at 95°C, and then 3 min at 65°C. Fresh-made mixture B was then added to the mixture A and incubated at 65°C for 75 min. Amplification products were analyzed on a 2% agarose gel (FIG. 2). A band of around 100 bp was observed in lane 2 (1% or 10 ng initial total RNA input) and lane 3 (0.1% or 1 ng initial total RNA input) on the agarose gel in agreement with the predicted size of 96 bp.

### Example 2: one-reaction vessel/two-buffer RT-HDA

In this example, the first-strand cDNA synthesis was coupled with tHDA reaction in one tube using two-step incubation. Different amount of human total RNA ranging from 10 pg to 100 ng and a pair of specific primers, GAPDF1 and GAPDR1, of the GAPDH gene were used for amplification.

The reaction with a total volume of 50 µl was set up in one tube by combining:
5 µl 10X Mg-free tHDA buffer
1 µl variable amount of human total RNA (10 pg - 100 ng)
0.5 µl 10 µM Primer GAPDF1 (SEQ ID NO:1)
0.5 µl 10 µM Primer GAPDR1 (SEQ ID NO:2)
1.75 µl 100 mM MgSO₄
2 µl 500 mM NaCl
2 µl 10 mM dNTP
1.5 µl 100 mM dATP
1 µl RNase inhibitor (10 units/µl)
1 µl M-MulV reverse transcriptase (25 units/µl)
2.5 µl Bst DNA polymerase, LF (8 units/µl, New England Biolabs, Inc., Ipswich, MA)
1 µl Tte-UvrD helicase (100 ng/µl)
30.25 µl nuclease-free H₂O

The RT-HDA reaction was carried out at 42°C for 5 min, followed by 65°C for 60 min. Amplification products were analyzed on a 2% agarose gel (FIG. 3). A strong band of around 100 bp was observed in the presence of 100 ng initial total RNA (lane 3) and 10 ng of initial total RNA (lane 4) on the agarose gel. A weak band of around 100 bp was also observed in the presence of 1 ng (lane 5) of initial total RNA on the agarose gel. The observed DNA fragment size was in agreement with the predicted target size of 96 bp. When the amount of input total RNA was below 1 ng, only none-specific amplification in the form of primer-dimer was observed (lanes 6 to 8). In addition, when reverse transcriptase (lane 1) or helicase (lane 2) was omitted from the reaction, no amplification was observed, suggesting the amplification depended on both RT and HDA functions.

### Example 3: one-reaction vessel/one-buffer RT-HDA

In this example, a reverse transcriptase with higher thermostability was selected and the first-strand cDNA synthesis and the helicase-dependent isothermal DNA amplification reaction were coupled and carried out in one tube and in one step. Different amount of human total RNA ranging from 100 pg to 100 ng and a pair of specific primers, GAPDF1 and GAPDR1, of the GAPDH gene were used for amplification.

The reaction with a total volume of 50 µl was set up in one tube by combining:
5 µl Mg-free tHDA buffer
1 µl variable amount of human total RNA (100 pg - 100 ng)
0.5 µl 10 µM Primer GAPDF1 (SEQ ID NO:1)
0.5 µl 10 µM Primer GAPDR1 (SEQ ID NO:2)
1.75 µl 100 mM MgSO₄
2 µl 500 mM NaCl
2 µl 10 mM dNTP
1.5 µl 100 mM dATP
1 µl SuperScript™ III reverse transcriptase (200 units/µl, Invitrogen, Carlsbad, CA)
2.5 µl Bst DNA polymerase, LF (8 units/µl, New England Biolabs, Inc., Ipswich, MA)
1 µl Tte-UvrD helicase (100 ng/µl)
31.25 µl nuclease-free H₂O

The RT-HDA reaction was carried out in one step at 62.5°C for 60 min. Amplification products were analyzed on a 2% agarose gel (FIG. 4). In the presence of 100 ng (lane 2) or 10 ng (lane 3) of initial total RNA, a DNA fragment of around 100 bp was observed on the agarose gel in agreement with the predicted target size of 96 bp. When the amount of input total RNA was below 10 ng, only none-specific amplification in the form of primer-dimer was observed (lanes 4 - 6).

### Example 4: modified one-buffer RT-HDA

In this example, a separate denaturation step involved in the denaturation of RNA template and primers was applied to decrease non-specific amplification. A reverse transcriptase with higher thermostability at 65°C was selected and the first-strand cDNA synthesis and the helicase-dependent isothermal DNA amplification reaction were coupled and carried out in one tube and in one step. Different amount of human total RNA ranging from 2 pg to 200 ng and a pair of specific primers, GAPDF3 and GAPDR3, located on different exons of the GAPDH gene were used for amplification.

The reaction with a total volume of 50 µl was set up by first making Mix A and Mix B. Mix A was prepared in one sterile microtube by combining:
2.5 µl 10X RT-HDA buffer (see below*)
2 µl variable amount of human total RNA (2 pg - 200 ng)
0.375 µl 10 µM primer GAPDF3 (SEQ ID NO:3)
0.375 µl 10 µM primer GAPDR3 (SEQ ID NO:4)
19.75 µl nuclease-free H₂O
Total volume: 25 µl

For the negative control reaction, replace 2 µl nuclease-free H₂O with the total RNA.

Mix B was prepared in another sterile microtube by combining:
2.5 µl 10X RT-HDA buffer (see below*)
1.75 µl 100 mM MgSO₄
4 µl 500 mM NaCl
2 µl 10 mM dNTP
1.5 µl 100 mM dATP
0.5 µl RNase inhibitor (40 units/µl, New England Biolabs, Inc., Ipswich, MA)
0.7 µl ThermoScript reverse transcriptase (15 units/µl, Invitrogen)
2.5 µl Bst DNA Polymerase, LF (8 units/µl, New England Biolabs, Inc., Ipswich, MA)
1 µl Tte-UvrD helicase (150 ng/µl)
8.55 µl nuclease-free H₂O
Total volume: 25 µl
*10X RT-HDA buffer contains 100 mM KCl, 200 mM Tris-HCl (pH 8.8 at 25°C).

Denaturation of Mix A was carried out at 95°C for 2 min. After cooling Mix A on ice, Mix B was added into Mix A. The RT-HDA reaction was carried out in one step at 65°C for 120 min. Amplification products were analyzed on a 2% agarose gel (FIG. 5). In the presence of as high as 200 ng down to as low as 2 pg of initial total RNA (lanes 2 - 7), a single DNA fragment of around 100 bp was observed on the agarose gel in agreement with the predicted target size of 95 bp. No none-specific amplification was observed from 2 ng to 200 ng of initial total RNA (lanes 2 - 7) or from the no template control (lane 8). This indicates that the modified one-step RT-HDA for detection of the GAPDH gene works well in a range of at least 5 log of total RNA.

### Example 5: detection of Enterovirus RNA

In this example, the modified one-step RT-HDA method described in Example 4 was applied in detection of a RNA virus Enterovirus. Enterovirus RNAs purified from the Enterovirus Clear QC Panel (Argene, Varilhes, France) ranging from 40 copies to 4000 copies were used as templates and a pair of specific primers, EVF1A and EVR1, targeting the 5' - UTR sequence conserved in all Enterovirus species were used as primers for amplification.

Purification of Enterovirus Clear QC Panel was carried out with a QIAamp Viral RNA Mini Kit (Qiagen, Valencia, CA). 140 µl of each Enterovirus standard sample with a concentration of 10 copies/µl, 100 copies/µl and 1000 copies/µl was used for purification and eluted into 70 µl of elution buffer. The modified one-step RT-HDA reaction with a total volume of 50 µl was set up by first making Mix A and Mix B. Mix A was prepared in one sterile microtube by combining:
2.5 µl 10X RT-HDA buffer (see below*)
2 µl variable amount of Enterovirus RNA (40 - 4000 copies)
0.375 µl 10 µM primer EVF1A (SEQ ID NO:5)
0.375 µl 10 µM primer EVR1 (SEQ ID NO:6)
19.75 µl nuclease-free H₂O
Total volume: 25 µl

For the negative control reaction, replace 2 µl nuclease-free H₂O with the Enterovirus RNA.

Mix B was prepared in another sterile microtube by combining:
2.5 µl 10X RT-HDA buffer (see below*)
1.75 µl 100 mM MgSO₄
2 µl 500 mM NaCl
2 µl 10 mM dNTP
1.5 µl 100 mM dATP
0.5 µl RNase inhibitor (40 units/µl, New England Biolabs, Inc., Ipswich, MA)
0.7 µl ThermoScript reverse transcriptase (15 units/µl, Invitrogen)
2.5 µl Bst DNA Polymerase, LF (8 units/µl, New England Biolabs, Inc., Ipswich, MA
1 µl Tte-UvrD helicase (150 ng/µl)
10.55 µl nuclease-free H₂O
Total volume: 25 µl
*10X RT-HDA buffer contains 100 mM KCl, 200 mM Tris-HCl (pH 8.8 at 25°C).

Denaturation of Mix A was carried out at 95°C for 2 min. After cooling Mix A on ice, Mix B was added into Mix A. The RT-HDA reaction was carried out in one step at 65°C for 120 min. Amplification products were analyzed on a 2% agarose gel (FIG. 6). In the presence of 4000 copies (lane 1) and 400 copies (lane 2) of initial Enterovirus RNA, a single DNA fragment of around 120 bp was observed on the agarose gel in agreement with the predicted target size of 116 bp. No amplification was observed from 40 copies of initial Enterovirus RNA (lane 3). None-specific amplification was not observed from 4000 copies to 40 copies of initial Enterovirus RNA (lanes 1 - 3) or from the no template control (lane 4).

## Claims

1. A method for helicase-dependent isothermal amplification of a RNA molecule in a single reaction vessel, the method comprising:
(a) adding to the RNA molecule, a mixture comprising a reverse transcriptase, a plurality of oligonucleotide primers, a polymerase, and a helicase preparation comprising a helicase, a nucleotide triphosphate and a single strand binding protein, wherein when the helicase is a thermostable helicase the mixture does not comprise a single strand binding protein;
(b) reverse transcribing the RNA to form an RNA/cDNA duplex and at least partially separating the duplex into a cDNA and an RNA using the helicase; and
(c) amplifying the cDNA by helicase-dependent amplification.

2. A method according to claim 1 wherein the reverse transcription (RT) in (b) results from a reverse transcriptase or a DNA polymerase with RT activity.

3. A method according to claim 1 wherein the isothermal amplification occurs at a temperature in the range of 20 to 75°C.

4. A method for identifying a RNA molecule comprising helicase-dependent isothermal amplification of the RNA in a single reaction vessel using a method comprising:
(a) adding to the RNA molecule, a mixture comprising a reverse transcriptase, a plurality of oligonucleotide primers, and one or more polymerase, and a helicase preparation comprising a helicase, a nucleotide triphosphate and a single strand binding protein, wherein when the helicase is a thermostable helicase the mixture does not comprise a single strand binding protein;
(b) reverse transcribing the RNA to form a RNA/cDNA duplex and at least partially separating the duplex into a cDNA and a RNA using the helicase;
(c) amplifying the cDNA by helicase-dependent amplification;
and
(d) characterizing the cDNA to determine the identity of the RNA molecule.

5. A method according to claim 4 wherein the isothermal amplification occurs at a temperature in the range of 20 to 75°C.

## Patentansprüche

1. Verfahren für helikaseabhängige isotherme Amplifikation eines RNA-Moleküls in einem einzelnen Reaktionsgefäß, wobei das Verfahren Folgendes umfasst:
(a) Hinzufügen einer Mischung zu dem RNA-Molekül, umfassend eine reverse Transkriptase, eine Vielzahl von Oligonukleotid-Primern, eine Polymerase und ein Helikasepräparat, umfassend eine Helikase, ein Nukleotidtriphosphat und ein einzelstrangbindendes Protein, wobei, wenn die Helikase eine thermostabile Helikase ist, die Mischung kein einzelstrangbindendes Protein umfasst;
(b) reverses Transkribieren der RNA, um einen RNA/cDNA-Duplex zu bilden, und zumindest teilweise Aufteilen des Duplex in eine cDNA und eine RNA unter Verwendung der Helikase; und
(c) Amplifizieren der cDNA durch helikaseabhängige Amplifikation.

2. Verfahren nach Anspruch 1, wobei die reverse Transkription (RT) in (b) aus einer reversen Transkriptase oder DNA-Polymerase mit RT-Aktivität resultiert.

3. Verfahren nach Anspruch 1, wobei die isotherme Amplifikation bei einer Temperatur im Bereich von 20 bis 75 °C auftritt.

4. Verfahren zum Identifizieren eines RNA-Moleküls, umfassend helikaseabhängige isotherme Amplifikation der RNA in einem einzelnen Reaktionsgefäß, unter Verwenden eines Verfahrens, das Folgendes umfasst:
(a) Hinzufügen einer Mischung zu dem RNA-Molekül, umfassend eine reverse Transkriptase, eine Vielzahl von Oligonukleotid-Primern und eine oder mehrere Polymerasen, und ein Helikasepräparat, umfassend eine Helikase, ein Nukleotidtriphosphat und ein einzelstrangbindendes Protein, wobei, wenn die Helikase eine thermostabile Helikase ist, die Mischung kein einzelstrangbindendes Protein umfasst;
(b) reverses Transkribieren der RNA, um einen RNA/cDNA-Duplex zu bilden, und zumindest teilweise Aufteilen des Duplex in eine cDNA und eine RNA unter Verwendung der Helikase;
(c) Amplifizieren der cDNA durch helikaseabhängige Amplifikation;
und
(d) Charakterisieren der cDNA, um die Identität des RNA-Moleküls zu bestimmen.

5. Verfahren nach Anspruch 4, wobei die isotherme Amplifikation bei einer Temperatur im Bereich von 20 bis 75 °C auftritt.

## Revendications

1. Méthode d'amplification isothermique dépendante d'une hélicase d'une molécule d'ARN dans un récipient de réaction unique, la méthode comprenant :
(a) l'ajout à la molécule d'ARN, d'un mélange comprenant une transcriptase inverse, une pluralité d'amorces oligonucléotidiques, une polymérase et une préparation d'hélicase comprenant une hélicase, un nucléotide triphosphaté et une protéine de liaison à un simple brin, dans laquelle lorsque l'hélicase est une hélicase thermostable le mélange ne comprend pas de protéine de liaison à un simple brin ;
(b) la transcription inverse de l'ARN pour former un duplex ARN/ADNc et au moins la séparation partielle du duplex en un ADNc et un ARN à l'aide d'une hélicase ; et
(c) l'amplification de l'ADNc avec une amplification dépendante d'une hélicase.

2. Méthode selon la revendication 1, dans laquelle la transcription inverse (TI) à l'étape (b) est réalisée grâce à une transcriptase inverse ou une ADN polymérase possédant une activité de TI.

3. Méthode selon la revendication 1, dans laquelle l'amplification isothermique se produit à une température dans la fourchette de 20 à 75 °C.

4. Méthode d'identification d'une molécule d'ARN comprenant une amplification isothermique dépendante d'une hélicase d'un ARN dans un récipient de réaction unique utilisant une méthode comprenant :
(a) l'ajout à la molécule d'ARN, d'un mélange comprenant une transcriptase inverse, une pluralité d'amorces oligonucléotidiques, et une ou plusieurs polymérases et une préparation d'hélicase comprenant une hélicase, un nucléotide triphosphaté et une protéine de liaison à un simple brin, dans laquelle lorsque l'hélicase est une hélicase thermostable le mélange ne comprend pas de protéine de liaison à un simple brin ;
(b) la transcription inverse de l'ARN pour former un duplex ARN/ADNc et au moins la séparation partielle du duplex en un ADNc et un ARN à l'aide d'une hélicase ;
(c) l'amplification de l'ADNc avec une amplification dépendante d'une hélicase ;
et
(d) la caractérisation de l'ADNc pour déterminer l'identité de la molécule d'ARN.

5. Méthode selon la revendication 4, dans laquelle l'amplification isothermique se produit à une température dans la fourchette de 20 à 75 °C.


### SEQUENCE LISTING

<110> Biohelix Corporation Kong, Huimin Tang, Wen
<120> Amplification of RNA Targets by Helicase-Dependent Amplification
<130> BH-002-PCT
<150> 60/641,419
   <151> 2005-01-05
<160> 6
<170> PatentIn version 3.2
<210> 1
   <211> 27
   <212> DNA
   <213> artificial
<220>
   <223> primer
<400> 1
   acatgttcca atatgattcc acccatg 27
<210> 2
   <211> 27
   <212> DNA
   <213> artificial
<220>
   <223> primer
<400> 2
   agatggtgat gggatttcca ttgatga 27
<210> 3
   <211> 27
   <212> DNA
   <213> artificial
<220>
   <223> primer
<400> 3
   actctggtaa agtggatatt gttgcca 27
<210> 4
   <211> 27
   <212> DNA
   <213> artificial
<220>
   <223> primer
<400> 4
   tttgccatgg gtggaatcat attggaa 27
<210> 5
   <211> 26
   <212> DNA
   <213> artificial
<220>
   <223> primer
<400> 5
   tcctccggcc cctgaatgcg gctaat 26
<210> 6
   <211> 26
   <212> DNA
   <213> artificial
<220>
   <223> primer
<400> 6
   cacggacacc caaagtagtc ggttcc 26